# EUROPÄISCHE PATENTSCHRIFT

(11) **EP 3 215 269 B1**
(45) Veröffentlichungstag und Bekanntmachung des Hinweises auf die Patenterteilung: **13.05.2020**
(21) Anmeldenummer: 15777687.3
(22) Anmeldetag: 07.10.2015
(51) Int. Cl.: C07C 37/06, C07C 39/15, B01J 23/656, B01J 23/63, B01J 35/08, B01J 35/10, B01J 21/04, B01J 37/18, B01J 37/02

(54) **VERFAHREN ZUR HERSTELLUNG VON ORTHO-PHENYLPHENOL (OPP)**
PROCESS FOR PRODUCING ORTHOPHENYLPHENOL (OPP)
PROCÉDÉ DE PRÉPARATION D'ORTHOPHÉNYLPHÉNOL (OPP)

(30) Priorität: 06.11.2014 EP 14192010
(43) Veröffentlichungstag der Anmeldung: 13.09.2017
(73) Patentinhaber: Lanxess Deutschland GmbH, 50569 Köln (DE)
(72) Erfinder: LARCHER, Christoph, 40476 Düsseldorf (DE); ZIRNGIEBL, Eberhard, 51061 Köln (DE); KURR, Patrick, 41061 Mönchengladbach (DE)
(86) Internationale Anmeldenummer: PCT/EP2015/073158
(87) Internationale Veröffentlichungsnummer: WO 2016/071066

(56) Entgegenhaltungen:
- DE-A1- 3 523 205
- DE-A1- 4 208 443
- US-A- 4 492 769

## Beschreibung

Die Erfindung betrifft die Herstellung von ortho-Phenylphenol unter Verwendung eines speziellen Trägerkatalysators.

Katalysatoren zur Herstellung von ortho-Phenylphenol (OPP, 2-Phenylphenol, 2-Hydroxybiphenyl) sowie deren Einsatz in Verfahren zur Herstellung von ortho-Phenylphenol sind beispielsweise aus DE 4132944 A1 und DE 3523205 A1 bekannt. Zwar zeigen diese Katalysatoren katalytische Aktivität, jedoch sind ihre Träger Chrom-haltig, da Ammoniumchromat, (NH₄)₂Cr₂O₇, zu ihrer Herstellung verwendet wurde. Chrom(VI) ist toxisch, kanzerogen und mutagen, wodurch hinsichtlich der Durchführung des Verfahrens hohe Anforderungen an die Produktionsanlagen und das Personal bestehen. Zudem sind Chrom(VI)-Verbindungen gemäß REACH-Verordnung als besonders besorgniserregende Stoffe auf der SVHC (Substances Of Very High Concern)-Liste aufgeführt. Daher besteht ein großes Bedürfnis nach einem vorzugsweise weniger als 100 ppm Chrom aufweisenden Katalysator für das Verfahren zur Herstellung von OPP.

In der DE 2211721 A1 wird auch ein Verfahren zur Herstellung von OPP offenbart. Der bei diesem Verfahren eingesetzte Katalysator wird hergestellt, indem Palladium, Platin, Iridium oder Rhodium oder ein Gemisch von zwei oder mehreren dieser Elemente auf ein Träger wie Kieselerde, Aluminiumoxid, Kieselerde-Aluminiumoxid oder Aktivkohle abgeschieden wird und dann eine geeignete Menge eines Alkalimetallsalzes hinzugegeben wird. Durch die Zugabe wird beabsichtigt, dass die sauren Stellen auf der Katalysatorträgeroberfläche verringert werden, welche gemäß DE 2211721 A1 als Beschleuniger für Zersetzungsreaktionen angesehen werden. Jedoch zeigen die Edelmetall-Katalysatoren, die in dem Verfahren nach DE 2211721 A1 zur Herstellung von OPP eingesetzt werden, eine unzureichende Aktivität und eine geringe Selektivität, insbesondere bei Einsatz eines Rhodium-haltigen Katalysators (S. 15, Tabelle 1, Beispiel 3, Selektivität 47 mol% OPP).

Somit war es die Aufgabe der vorliegenden Erfindung, einen Katalysator zur Herstellung von OPP bereitzustellen, der vorzugsweise eine hohe Aktivität sowie eine hohe Selektivität in dem Verfahren zur Herstellung von OPP aufweist. Des Weiteren soll der Katalysator vorzugsweise weniger als 100 ppm Chrom aufweisen. Des Weiteren soll der Katalysator für die großtechnische Herstellung von OPP geeignet sein. Vorzugsweise soll unter Verwendung des Katalysators das bei der Herstellung von OPP gängige, jedoch schwer abtrennbare Nebenprodukt Diphenyl nur in geringer Menge entstehen, bevorzugt zu weniger als 1.5 Gew.-%.

Es wurde nun ein Verfahren zur Herstellung von OPP durch Dehydrierung von von wenigstens einer Verbindung der Komponente A, die aus vollständig oder teilweise hydriertem OPP besteht, das dadurch gekennzeichnet ist, dass ein Trägerkatalysator als Katalysator eingesetzt wird, der Rhodium, Mangan, Cer und einen Träger enthält und weniger als 100 ppm Chrom, bezogen auf das Gesamtgewicht des Trägerkatalysators, enthält eingesetzt.

Bevorzugt enthält der Trägerkatalysator
- 0,1 - 5,0 Gew.-%, besonders bevorzugt 0,3 - 2,5 Gew.-%, ganz besonders bevorzugt 0,5 - 1,5 Gew.-% Rhodium,
- 0,025 - 5,0 Gew.-%, besonders bevorzugt 0,1 - 3,0 Gew.-%, ganz besonders bevorzugt 1,0 - 2,5 Gew.-% Mangan und
- 0,025 - 5,0 Gew.-%, besonders bevorzugt 0,1 - 3,0 Gew.-%, ganz besonders bevorzugt 1,5 - 2,5 Gew.-% Cer,
bezogen auf das Gesamtgewicht des Trägerkatalysators.

Das Gewichtsverhältnis der Metalle Rhodium, Mangan und Cer kann über einen weiten Bereich variieren. Bevorzugt beträgt das Gewichtsverhältnis von Mangan : Cer = 5 : 1 bis 1 : 5, besonders bevorzugt 9 : 10 bis 2 : 1.

Die Metalle Rhodium, Mangan und Cer können in jeglicher Form, beispielsweise in Form des jeweiligen Metalls in der Oxidationsstufe = 0, in Form von Verbindungen wie Oxiden, Sulfaten, Chloriden, Acetaten, Nitraten oder Gemischen davon, auf dem Träger vorliegen. Im Rahmen dieser Erfindung beinhalten die Begriffe "Rhodium", "Mangan" und "Cer" nicht zwingend, dass das jeweilige Metall elementar, d.h. in der Oxidationsstufe = 0 vorliegt, sofern nicht anderes beschrieben.

In einer bevorzugten Ausführungsform enthält der Trägerkatalysator Rhodium in Form von Rhodiumtrichlorid.

In einer anderen bevorzugten Ausführungsform enthält der Trägerkatalysator katalytisch aktives Rhodium.

In einer besonders bevorzugten Ausführungsform des Trägerkatalysators liegen wenigstens 50 Gew.-%, besonders bevorzugt wenigstens 70 Gew.-%, ganz besonders bevorzugt wenigstens 90 Gew.-% des Rhodiums in dem Trägerkatalysator in der Oxidationsstufe = 0 vor.

Die Verbindung zwischen den Metallen Rhodium, Mangan und Cer und dem Träger ist beliebig, beispielsweise kovalent, ionisch, adsorptiv oder Gemische davon.

Die Verteilung der Metalle Rhodium, Mangan und Cer auf dem Träger ist beliebig. Bevorzugt sind die Metalle über die gesamte Oberfläche des Trägers verteilt.

Der eingesetzte Trägerkatalysator enthält bevorzugt weniger als 50 ppm, ganz besonders bevorzugt weniger als 20 ppm Chrom, bezogen auf das Gesamtgewicht des Trägerkatalysators.

### Träger

Als Träger des eingesetzten Trägerkatalysators kommen die üblichen Katalysatorträgermaterialien in Frage. Bevorzugt ist der Träger ausgewählt aus der Gruppe bestehend aus α-, γ-, θ-, η- und δ- Aluminiumoxiden (Al₂O₃), Magnesium/Aluminium-Mischoxiden, Kieselgel, Kieselgur, Montmorilloniten, Bimsstein und Aktivkohle, bevorzugt aus der Gruppe bestehend aus γ- Aluminiumoxid und Magnesium/Aluminium-Mischoxiden.

Die BET-Oberfläche des Trägers beträgt bevorzugt 100 - 450 m²/g, besonders bevorzugt 160 - 350 m²/g, ganz besonders bevorzugt 230 - 300 m²/g.

Unter BET-Oberfläche wird die spezifische Oberfläche des Trägers verstanden, die in der vorliegenden Erfindung gemäß der DIN ISO 9277 ermittelt wurde.

Der eingesetzte Trägerkatalysator enthält bevorzugt einen kugelförmigen Träger, besonders bevorzugt mit einem Durchmesser von 1 - 5 mm, ganz besonders bevorzugt von 2 - 5 mm, ganz ganz besonders bevorzugt von 2-4 mm.

Der Natrium-Gehalt des Trägers kann über einen weiten Bereich variieren. Bevorzugt beträgt der Natrium-Gehalt des Trägers bis zu 3000 ppm, besonders bevorzugt bis zu 2600 ppm, bezogen auf das Gesamtgewicht des Trägers.

In einer bevorzugten Ausführungsform beträgt der Natrium-Gehalt des Trägers 1850 - 3000 ppm, besonders bevorzugt 2000 - 2600 ppm, bezogen auf das Gesamtgewicht des Trägers.

In einer weiteren bevorzugten Ausführungsform beträgt der Natrium-Gehalt des Trägers weniger als 800 ppm, besonders bevorzugt weniger als 650 ppm, bezogen auf das Gesamtgewicht des Trägers.

In einer bevorzugten Ausführungsform der vorliegenden Erfindung enthält der Trägerkatalysator γ-Aluminiumoxid (γ-Al₂O₃) als Träger.

Die BET-Oberfläche des γ-Al₂O₃ beträgt bevorzugt 150 - 450 m²/g, besonders bevorzugt 200 - 350 m²/g, ganz besonders bevorzugt 230 - 300 m²/g.

Der eingesetzte Trägerkatalysator enthält bevorzugt kugelförmiges γ-Al₂O₃, besonders bevorzugt mit einem Durchmesser von 1 - 5 mm, ganz besonders bevorzugt von 2 - 5 mm, ganz ganz besonders bevorzugt von 2-4 mm.

Besonders bevorzugt enthält der eingesetzte Trägerkatalysator kugelförmiges γ-Al₂O₃ als Träger mit einer BET-Oberfläche von 230 - 300 m²/g und einem Durchmesser von 2-4 mm.

Der Natrium-Gehalt des Trägers γ-Aluminiumoxid (γ-Al₂O₃) kann über einen weiten Bereich variieren.

In einer bevorzugten Ausführungsform beträgt der Natrium-Gehalt des Trägers γ-Aluminiumoxid 1850 - 3000 ppm, besonders bevorzugt 2000 - 2600 ppm, bezogen auf das Gesamtgewicht des Trägers. Es gelten entsprechend die vorherigen Beschreibungen und Vorzugsbereiche für γ-Aluminiumoxid (γ-Al₂O₃).

In einer weiteren bevorzugten Ausführungsform beträgt der Natrium-Gehalt des Trägers γ-Aluminiumoxid weniger als 800 ppm, besonders bevorzugt weniger als 650 ppm, bezogen auf das Gesamtgewicht des Trägers. Es gelten entsprechend die vorherigen Beschreibungen und Vorzugsbereiche für γ-Aluminiumoxid (γ-Al₂O₃).

In einer anderen bevorzugten Ausführungsform der vorliegenden Erfindung enthält der Trägerkatalysator Magnesium/Aluminium-Mischoxide als Träger, besonders bevorzugt enthaltend 5-10 Gew.-%, ganz besonders bevorzugt 6-8 Gew.-% Magnesiumoxid (MgO), bezogen auf das Gesamtgewicht des Trägers.

Die BET-Oberfläche des Magnesium/Aluminium-Mischoxids beträgt bevorzugt 100 - 300 m²/g, besonders bevorzugt 160 - 220 m²/g.

Der eingesetzte Trägerkatalysator enthält bevorzugt kugelförmiges Magnesium/Aluminium-Mischoxid, besonders bevorzugt mit einem Durchmesser von 1 - 3,5 mm, ganz besonders bevorzugt von 2-3 mm.

### Weitere Zusatzstoffe

Des Weiteren kann der eingesetzte Trägerkatalysator weitere Zusatzstoffe enthalten oder nicht enthalten.

Bevorzugt enthält der verwendete Trägerkatalysator wenigstens einen weiteren Zusatzstoff.

Als weitere Zusatzstoffe kommen beispielsweise Alkalimetalle, Erdalkalimetalle, Schwefel sowie Halogenide in Frage.

Bevorzugt enthält der verwendete Trägerkatalysator als wenigstens einen weiteren Zusatzstoff Alkalimetall, besonders bevorzugt ausgewählt aus Natrium, Kalium und Gemischen davon.

Besonders bevorzugt enthält der Trägerkatalysator als wenigstens einen weiteren Zusatzstoff wenigstens Alkalimetall und Schwefel.

Bevorzugte Mengen des Alkalimetalls in dem Trägerkatalysator sind 0,05 - 15 Gew.-%, besonders bevorzugt 0,1 - 10 Gew.-%, bezogen auf das Gesamtgewicht des Trägerkatalysators.

Bevorzugte Mengen des Schwefels in dem n Trägerkatalysator sind 0,05 - 3,0 Gew.-%, besonders bevorzugt 0,1 - 2,5 Gew.-%, bezogen auf das Gesamtgewicht des Trägerkatalysators.

### Verfahren zur Herstellung des Trägerkatalysators

Ein Verfahren zur Herstellung des Trägerkatalysators, ist Bspw. gekennzeichnet durch die Schritte
a) Aufbringen von Cer und Mangan auf einen Träger,
b) Erhitzen des unter Schritt a) erhaltenen mit Cer und Mangan beaufschlagten Trägers auf 200 - 500 °C,
c) Aufbringen von Rhodium auf den nach Schritt b) erhaltenen mit Cer und Mangan beaufschlagten Träger, und optional
d) Aufbringen wenigstens eines Alkalimetalls auf den nach Schritt c) erhaltenen mit Cer, Mangan und Rhodium beaufschlagten Träger.

Bevorzugt enthält das Verfahren zur Herstellung des Trägerkatalysators die Schritte a), b), c) und d).

Wie bereits weiter oben zu dem Trägerkatalysator erwähnt, beinhalten auch in Bezug auf das erfindungsgemäße Verfahren zur Herstellung des Trägerkatalysators die Begriffe "Rhodium", "Mangan" und "Cer" im Rahmen dieser Erfindung nicht zwingend, dass das jeweilige Metall elementar, d.h. in der Oxidationsstufe = 0 vorliegt, sofern nicht anderes beschrieben. Abhängig davon, in welcher Verbindung das jeweilige Metall in dem jeweiligen Verfahrensschritt auf den jeweiligen Träger aufgebracht wurde bzw. welche weitere Umsetzung in dem jeweiligen Verfahrensschritt durchgeführt wurde, kann abgeleitet werden, in welcher Form das jeweilige Metall nach dem jeweiligen Verfahrensschritt auf dem Träger vorliegt.

### Schritt a)

Das Aufbringen des Cers und des Mangans auf einen Träger in Schritt a) kann durch dem Fachmann bekannte Methoden erfolgen, z. B.
- durch gemeinsames Ausfällen eines Cer-Mangan-Hydroxidgemisches aus einer Cer- und Mangansalz-Lösung mit Alkalilauge oder Ammoniak und anschließendem Auswaschen der löslichen Anteile mit Wasser,
- durch Vermischen einer wässrigen Cer- und Mangansalz-Lösung mit dem Träger, oder
- durch Tränken oder Sprühen mit geeigneten Salz-Lösungen des Cers und des Mangans.

Bevorzugt erfolgt das Aufbringen in Schritt a) durch Vermischen einer wässrigen Cer- und Mangansalz-Lösung mit dem Träger. Als Cer- und Mangansalze kommen bevorzugt Sulfate, Chloride, Acetate und/oder Nitrate der genannten Elemente in Betracht. Besonders bevorzugt erfolgt das Aufbringen in Schritt a) durch Vermischen einer wässrigen Cer- und Mangannitrat-Lösung mit dem Träger.

Für den in Schritt a) eingesetzten Träger gelten alle vorherigen Beschreibungen und Vorzugsbereiche.

Bevorzugt wird der mit Cer und Mangan beaufschlagte Träger erhalten nach Schritt a) getrocknet. Besonders bevorzugt erfolgt die Trocknung bei 100 - 150 °C, ganz besonders bevorzugt bei 110 - 130 °C. Der Fachmann kann die geeignete Dauer der Trocknung ermitteln. Die Dauer der Trocknung beträgt bevorzugt 1 - 25 Stunden, besonders bevorzugt 10 - 20 Stunden.

### Schritt b)

Das Erhitzen des unter Schritt a) erhaltenen mit Cer und Mangan beaufschlagten Trägers in Schritt b) erfolgt auf 200 - 500 °C, bevorzugt auf 350 - 450 °C, besonders bevorzugt auf 380 - 420 °C. Die Dauer des Erhitzens beträgt bevorzugt 1 - 5 Stunden, besonders bevorzugt 2 - 4 Stunden.

Sofern in Schritt a) eine wässrige Cer- und Mangannitrat-Lösung eingesetzt wurde, erfolgt während des Erhitzens eine Abspaltung von Stickoxiden.

### Schritt c)

Das Aufbringen des Rhodiums auf den nach Schritt b) erhaltenen mit Cer und Mangan beaufschlagten Träger erfolgt nach dem Fachmann bekannten Methoden, bevorzugt durch Aufsprühen einer wässrigen Rhodiumsalz-Lösung auf den nach Schritt b) erhaltenen mit Cer und Mangan beaufschlagten Träger oder durch Vermischen einer wässrigen Rhodiumsalz-Lösung mit dem nach Schritt b) erhaltenen mit Cer und Mangan beaufschlagten Träger.

Besonders bevorzugt erfolgt das Aufbringen des Rhodiums in Schritt b) durch Vermischen einer wässrigen Rhodiumsalz-Lösung mit dem nach Schritt b) erhaltenen mit Cer und Mangan beaufschlagten Träger.

Als wässrige Rhodiumsalz-Lösung kommen beispielsweise wässrige Rhodiumtrichlorid-, wässrige Rhodiumnitrat- oder wässrige Rhodiumacetat-Lösungen in Frage. Bevorzugt wird eine wässrige Rhodiumtrichlorid-Lösung in Schritt c) eingesetzt.

Bevorzugt erfolgt das Vermischen der wässrigen Rhodiumsalz-Lösung mit dem nach Schritt b) erhaltenen mit Cer und Mangan beaufschlagten Träger in Form einer Tränkung.

Unter Tränkung eines Trägers wird im Rahmen der vorliegenden Erfindung das Vollsaugen des zu tränkenden Trägers mit einer entsprechenden wässrigen Lösung verstanden.

Bevorzugt wird der mit Cer, Mangan und Rhodium beaufschlagte Träger erhalten nach Schritt c) getrocknet. Besonders bevorzugt erfolgt die Trocknung bei 100 - 150 °C, ganz besonders bevorzugt bei 110 - 130 °C. Der Fachmann kann die geeignete Dauer der Trocknung ermitteln. Die Dauer der Trocknung beträgt bevorzugt 5-25 Stunden, besonders bevorzugt 10 - 20 Stunden.

### Schritt d)

Das offenbarte Verfahren kann Schritt d) enthalten oder nicht enthalten. Bevorzugt enthält das Verfahren Schritt d).

Das Aufbringen wenigstens eines Alkalimetalls auf den nach Schritt c) erhaltenen mit Cer, Mangan und Rhodium beaufschlagten Träger erfolgt in Schritt d) bevorzugt durch Vermischen des nach Schritt c) erhaltenen mit Cer, Mangan und Rhodium beaufschlagten Trägers mit einer wässrigen Lösung von wenigstens einer Alkalimetallverbindung.

Als wenigstens eine Alkalimetallverbindung kann eine schwefelfreie Alkalimetallverbindung, eine schwefelhaltige Alkalimetallverbindung oder ein Gemisch davon in Schritt d) eingesetzt werden.

Unter schwefelfreie Alkalimetallverbindung wird im Rahmen dieser Erfindung verstanden, dass das Gegenion des Alkalimetalls in der Alkalimetallverbindung kein Schwefel-Ion ist oder enthält.

Unter schwefelhaltige Alkalimetallverbindung wird im Rahmen dieser Erfindung verstanden, dass das Gegenion des Alkalimetalls in der Alkalimetallverbindung ein Schwefel-Ion ist oder enthält.

Beispiele für schwefelfreie Alkalimetallverbindungen sind Alkalimetalloxide, Alkalimetallhydroxide, Alkalimetallcarbonate, Alkalimetallbicarbonate, Alkalimetallacetate und Alkalialkoholate sowie Alkalimetallsalze niederer Carbonsäuren sowie Gemische aus den zuvor genannten.

Bevorzugte schwefelfreie Alkalimetallverbindungen sind Lithiumhydroxid, Natriumhydroxid, Natriumcarbonat, Natriumhydrogencarbonat, Natriummethylat, Natriumethylat, Natriumacetat, Kaliumhydroxid, Kaliumcarbonat, Kaliummethylat, Rubidiumhydroxid und Gemische aus den zuvor genannten, besonders bevorzugt Natriumhydroxid.

Beispiele für schwefelhaltige Alkalimetallverbindungen sind Alkalimetallsulfate, -sulfite, -thiosulfate und -thiocyanate, bevorzugt Kaliumsulfat, Natriumsulfat, Diatriumdisulfit, Natriumsulfit, Kaliumthiocyanat, Natriumthiocyanat und Gemische aus den zuvor genannten, besonders bevorzugt Kaliumsulfat.

Bevorzugt wird in Schritt d) eine wässrige Lösung von wenigstens einer Alkalimetallverbindung, welche ein Gemisch aus einer schwefelhaltigen und einer schwefelfreien Alkalimetallverbindung ist, mit dem nach Schritt c) erhaltenen mit Cer, Mangan und Rhodium beaufschlagten Träger vermischt. Besonders bevorzugt wird in Schritt d) eine wässrige Lösung von Natriumhydroxid und Kaliumsulfat mit dem nach Schritt c) erhaltenen mit Cer, Mangan und Rhodium beaufschlagten Träger vermischt.

Bevorzugt erfolgt das Vermischen des nach Schritt c) erhaltenen mit Cer, Mangan und Rhodium beaufschlagten Trägers mit einer wässrigen Lösung von wenigstens einer Alkalimetallverbindung, welche ein Gemisch aus einer schwefelhaltigen und einer schwefelfreien Alkalimetallverbindung ist, in Form einer Tränkung.

Die Konzentration der wenigstens einen Alkalimetallverbindung in der in Schritt d) eingesetzten wässrigen Lösung von wenigstens einer Alkalimetallverbindung beträgt bevorzugt 0,02 - 5 molar, besonders bevorzugt 0,1 - 4 molar, ganz besonders bevorzugt 0,5 - 3 molar.

Die Konzentration der schwefelfreien Alkalimetallverbindung der wenigstens einen Alkalimetallverbindung in der in Schritt d) eingesetzten wässrigen Lösung von wenigstens einer Alkalimetallverbindung beträgt bevorzugt 0,02 - 5 molar, besonders bevorzugt 0,1 - 4 molar, ganz besonders bevorzugt 0,5 - 3 molar.

Die Konzentration der schwefelhaltigen Alkalimetallverbindung der wenigstens einen Alkalimetallverbindung in der in Schritt d) eingesetzten wässrigen Lösung von wenigstens einer Alkalimetallverbindung beträgt bevorzugt 0,02 - 5 molar, besonders bevorzugt 0,1 - 4 molar, ganz besonders bevorzugt 0,1 - 2 molar.

Bevorzugt wird der mit Cer, Mangan, Rhodium und wenigstens einem Alkalimetall beaufschlagte Träger erhalten nach Schritt d) getrocknet. Besonders bevorzugt erfolgt die Trocknung bei 100 - 150 °C, ganz besonders bevorzugt bei 110 - 130 °C. Der Fachmann kann die geeignete Dauer der Trocknung ermitteln. Die Dauer der Trocknung beträgt bevorzugt solange, bis eine Ablufttemperatur von 100 - 120 °C erreicht ist.

Bevorzugt werden die Schritte a), b) c) und d) in der gelisteten Reihenfolge hintereinander durchgeführt.

Die nach Schritt c) und alternativ nach Schritt d) erhaltenen Trägerkatalysatoren wiesen eine ausgezeichnete Stabilität hinsichtlich des Transports auf.

### Schritt e)

In dem Verfahren zur Herstellung des Trägerkatalysators kann sich dem Schritt c) oder, sofern Schritt d) in dem Verfahren enthalten ist, dem Schritt d) der Schritt e) anschließen oder nicht anschließen.

Schritt e) ist gekennzeichnet durch:
e) Umsetzung des nach Schritt c) erhaltenen mit Cer, Mangan und Rhodium beaufschlagten Trägers, bevorzugt des nach Schritt d) erhaltenen mit Cer, Mangan, Rhodium und wenigstens einem Alkalimetall beaufschlagten Trägers, mit Wasserstoff.

Die Umsetzung mit Wasserstoff erfolgt bevorzugt bei 120 - 450 °C, besonders bevorzugt bei 350 - 420 °C. Bevorzugt erfolgt das Behandeln des Trägerkatalysators mit Wasserstoff für 15 - 80 Stunden.

Durch die Umsetzung in Schritt e) entsteht in dem Trägerkatalysator katalytisch aktives Rhodium.

Bevorzugt liegen in dem Trägerkatalysator erhalten nach Schritt e) wenigstens 50 Gew.-%, besonders bevorzugt wenigstens 70 Gew.-%, ganz besonders bevorzugt wenigstens 90 Gew.-% des Rhodiums in der Oxidationsstufe = 0 vor.

Der nach Schritt e) erhaltene Trägerkatalysator eignet sich daher bevorzugt zur Herstellung von OPP.

Herstellung von ortho-Phenylphenol (OPP)lm Rahmen der vorliegenden Erfindung beziehen sich die Begriffe "vollständig hydriert" und "teilweise hydriert" in Bezug auf die Verbindung der Komponente A, die aus vollständig oder teilweise hydriertem OPP besteht, auf den Zustand der Doppelbindungen der aromatischen Sechsringe von OPP. Unter einem teilweise hydrierten OPP wird im Rahmen der vorliegenden Erfindung ein OPP-Derivat verstanden, in dem wenigstens eine, jedoch nicht alle der Doppelbindungen der aromatischen Sechsringe von OPP hydriert ist. Unter einem vollständig hydrierten OPP wird im Rahmen der vorliegenden Erfindung ein OPP-Derivat verstanden, in dem alle Doppelbindungen der aromatischen Sechsringe von OPP hydriert sind.

Die wenigstens eine Verbindung der Komponente A, die aus vollständig oder teilweise hydriertem OPP besteht, ist bevorzugt ausgewählt aus der Gruppe bestehend aus 2-Cyclohexylidencyclohexanon, 2-Cyclohexenylcyclohexanon, 2-Cyclohexylcyclohexanon, 2-Cyclohexylcyclohexanol, 2-Cyclohexylphenol, 3-Cyclohexylphenol, 4-Cyclohexylphenol, 2-Phenylcyclohexanon, 2-Phenylcyclohexanol und Gemischen davon.

Besonders bevorzugt ist die wenigstens eine Verbindung der Komponente A ein Gemisch aus 2-Cyclohexylidencyclohexanon und 2-Cyclohexenylcyclohexanon.

Die bevorzugten Verbindungen der Komponente A sind auf dem Fachmann bekannte Weisen zugänglich. So erhält man beispielsweise 2-Cyclohexylidencyclohexanon und 2-Cyclohexenylcyclohexanon durch Kondensieren von Cyclohexanon in Gegenwart saurer oder basischer Katalysatoren nach bekannten Methoden. Diese beiden Verbindungen entstehen außerdem neben 2-Cyclohexylcyclohexanon und 2-Cyclohexylcyclohexanol u. a. als Nebenprodukte bei der katalytischen Cyclohexanol-Dehydrierung. Sie können aus dem Dehydriergemisch leicht destillativ abgetrennt und als Gemisch für die OPP-Herstellung verwandt werden.

Der obige Trägerkatalysator kann direkt zur katalytischen Dehydrierung eingesetzt werden.

Bevorzugt wird in dem erfindungsgemäßen Verfahren zur Herstellung von OPP ein Trägerkatalysator eingesetzt, der katalytisch aktives Rhodium enthält.

Besonders bevorzugt wird in dem erfindungsgemäßen Verfahren zur Herstellung von OPP ein Trägerkatalysator eingesetzt, in dem wenigstens 50 Gew.-%, besonders bevorzugt wenigstens 70 Gew.-%, ganz besonders bevorzugt wenigstens 90 Gew.-% des Rhodiums in der Oxidationsstufe = 0 vorliegen.

Ganz besonders bevorzugt wird der in dem erfindungsgemäßen Verfahren zur Herstellung von OPP eingesetzte Trägerkatalysator, der bevorzugt katalytisch aktives Rhodium enthält, wobei besonders bevorzugt wenigstens 50 Gew.-%, ganz besonders bevorzugt wenigstens 70 Gew.-%, ganz ganz besonders bevorzugt wenigstens 90 Gew.-% des Rhodiums in der Oxidationsstufe = 0 vorliegen, durch Umsetzung eines mit Cer, Mangan und Rhodium beaufschlagten Trägers, bevorzugt mit Cer, Mangan, Rhodium und wenigstens einem Alkalimetall beaufschlagten Trägers, mit Wasserstoff erhalten.

In einer bevorzugten Ausführungsform des erfindungsgemäßen Verfahrens zur Herstellung von OPP ist die katalytische Dehydrierung dadurch charakterisiert, dass die wenigstens eine Verbindung der Komponente A, die aus vollständig oder teilweise hydriertem OPP besteht, und der Trägerkatalysator bei einer Temperatur von 300 - 450 °C, bevorzugt 330 - 400 °C, in Gegenwart von Wasserstoff und Stickstoff umgesetzt werden.

Bevorzugt ist die katalytische Dehydrierung des vorliegenden erfindungsgemäßen Verfahrens zur Herstellung von OPP eine Gasphasendehydrierung.

Bevorzugt beträgt das Volumenverhältnis von Wasserstoff zu Stickstoff in der katalytischen Dehydrierung 10:1 -1:15, besonders bevorzugt 5:1 -1:5.

In einer besonders bevorzugten Ausführungsform ist die katalytische Dehydrierung dadurch charakterisiert, dass zunächst der Trägerkatalysator auf eine Temperatur von 300 - 450 °C, bevorzugt 330 - 400 °C, erhitzt wird und im Anschluss die wenigstens eine Verbindung der Komponente A, die aus vollständig oder teilweise hydriertem OPP besteht, gemeinsam mit Wasserstoff und Stickstoff zu dem Trägerkatalysator gefügt wird.

Bevorzugt werden Wasserstoff und Stickstoff in Form eines Stroms über den Trägerkatalysator geleitet.

Das nach dem erfindungsgemäßen Verfahren hergestellte OPP kann als Konservierungsmittel, beispielsweise für Citrusfrüchte oder in Desinfektionsmitteln, eingesetzt werden.

Anhand der nachfolgenden Beispiele wird die Erfindung näher erläutert, ohne dass dadurch eine Einschränkung der Erfindung bewirkt werden soll.

### Beispiele

### Beispiel 1

### Beispiel 1a - Herstellung des Trägerkatalysators

2120 g eines handelsüblichen kugelförmigen γ-Al₂O₃ mit 2 - 4 mm Durchmesser, mit einer BET-Oberfläche von 230 - 300 m²/g und einem Natriumgehalt von ca. 520 ppm wurden mit 1,17 Litern einer wässrigen Lösung von 131,44 g Ce(NO₃)₃ x 6 H₂O und 193,77 g Mn(NO₃)₂ x 4 H₂O so lange gerührt, bis die Lösung komplett aufgesaugt war, im Anschluss 18 Stunden bei 120 °C im Warmlufttrockner getrocknet und schließlich 3 Stunden bei 400 °C erhitzt.

1868 g des nach dem Erhitzen erhaltenen, mit Cer und Mangan beaufschlagten Trägers wurden mit einer Lösung von 93,3 g RhCl₃ x H₂O in 0,850 Liter destilliertem Wasser gleichmäßig getränkt. Der mit Cer, Mangan und Rhodium beaufschlagte Träger wurde im Warmlufttrockner bei 120 °C getrocknet.

59,8 g NaOH und 59,8 g K₂SO₄ wurden in 500 ml destilliertem Wasser gelöst und auf 780 ml Gesamtvolumen mit destilliertem Wasser aufgefüllt. Mit dieser Lösung wurden 1950 g des mit Cer, Mangan und Rhodium beaufschlagte Trägers gleichmäßig getränkt und abschließend im Warmlufttrockner bei 120 °C getrocknet bis eine Ablufttemperatur von 110 °C erreicht war.

Der erhaltene Trägerkatalysator enthielt 0,75 Gew.-% Rhodium, 1,63 Gew.-% Mangan und 2,04 Gew.-% Cer, bezogen auf das Gesamtgewicht des Trägerkatalysators.

Der Chrom-Gehalt des Trägerkatalysators betrug weniger als 20 ppm, bezogen auf das Gesamtgewicht des Trägerkatalysators (ermittelt mittels Röntgenfluoreszenzanalyse, Spektrometer-Software SpectraPlus, S8, Bruker AXS, Karlsruhe, Deutschland).

### Beispiel 1b - Umsetzung des Trägerkatalysators mit Wassersoff

50 ml (42,2 g) des Trägerkatalysators aus Beispiel 1a wurden in einem senkrecht stehenden, elektrisch beheizten Stahlrohr von 120 cm Länge und 14,1 mm Innendurchmesser eingebracht. Es erfolgte eine Reduktion des Trägerkatalysators aus Beispiel 1a bei 385 °C in einem Wasserstoffstrom von bis zu 10 L/h für 22 Stunden.

### Beispiel 1c - Verfahren zur Herstellung von OPP unter Verwendung des Trägerkatalysators

Der Reaktor aus Beispiel 1b enthaltend den Trägerkatalysator erhalten nach Beispiel 1b wurde auf 330 - 400 °C erhitzt und unter Benutzung einer geeichten Dosiervorrichtung wurden 0,25 ml/min eines Gemischs von 2-Cyclohexenylcyclohexanon und 2-Cyclohexylidencyclohexanon zusammen mit 3 L H₂ und 5 L N₂ pro Stunde in das Reaktionsrohr geleitet. Im oberen Teil des Rohres, in dem sich nur Füllkörper befanden, erfolgte die Verdampfung des flüssigen Ausgangsgemisches.

Nach 267 Stunden wies das Reaktionsprodukt bei 400 °C Katalysatortemperatur folgende Zusammensetzung auf:

| | |
|---|---|
| ortho-Phenylphenol (OPP): | 85 % |
| Diphenyloxid: | 5.1 % |
| Diphenyl: | 0.8% |
| Phenol: | 0.2 % |

### Beispiel 1d (Vergleichsbeispiel)

Die Durchführung erfolgte analog Beispiel 1b und 1c, mit der Änderung, dass in Beispiel 1b 50 ml (49,8 g) des Chrom-, Mangan- und Rhodium-haltigen Trägerkatalysators erhalten nach Beispiel 1a der Anmeldung DE 3523205 A1 statt des Trägerkatalysators eingesetzt wurden.

Nach 267 Stunden wies das Reaktionsprodukt bei 400 °C Katalysatortemperatur folgende Zusammensetzung auf:

| | |
|---|---|
| ortho-Phenylphenol (OPP): | 75 % |
| Diphenyloxid: | 3.6 % |
| Diphenyl: | 1.7 % |
| Phenol: | 0.7 % |

Die Ausbeute an OPP ist in Beispiel 1d mit 75 % deutlich geringer als in Beispiel 1c unter Verwendung des Trägerkatalysators. Des Weiteren ist der erhaltene Anteil an unerwünschten Nebenproduktes Diphenyl deutlich höher als in Beispiel 1c unter Verwendung des Trägerkatalysators.

Die Ausbeute an OPP ist unter den Reaktionsbedingungen in Beispiel 1d (Vergleichsbeispiel) der vorliegenden Anmeldung geringer als unter den Reaktionsbedingungen in Beispiel 1b der Anmeldung DE 3523205 A1, da aufgrund des in der vorliegenden Anmeldung verwendeten längeren und schmaleren Rohres eine höhere Flächenbelastung in diesem Rohr vorherrscht verglichen mit der Flächenbelastung des in Beispiel 1b der DE 3523205 A1 verwendeten Rohres. Diese erhöhte Flächenbelastung führt bei endothermen Reaktionen wie der vorliegenden Dehydrierung zu einer geringeren Ausbeute an OPP. Die Reaktionsbedingungen der vorliegenden Erfindung simulieren die großtechnische Herstellung jedoch deutlich besser, da großtechnisch Rohrbündelreaktoren mit einer Länge von mehreren Metern und einem geringem Durchmesser der Einzelrohre eingesetzt werden.

### Beispiel 2

### Beispiel 2a - Herstellung des Trägerkatalysators

1000 g eines handelsüblichen kugelförmigen γ-Al₂O₃ mit 2 - 5 mm Durchmesser, mit einer BET-Oberfläche von 320 - 335 m²/g und einem Natriumgehalt von ca. 2370 ppm wurden mit 410 ml einer wässrigen Lösung von 62,0 g Ce(NO₃)₃ x 6 H₂O und 91,4 g Mn(NO₃)₂ x 4 H₂O so lange gerührt, bis die Lösung komplett aufgesaugt war, im Anschluss 18 Stunden bei 120 °C im Warmlufttrockner getrocknet und schließlich 3 Stunden bei 400 °C erhitzt.

186 g des nach dem Erhitzen erhaltenen, mit Cer und Mangan beaufschlagten Trägers wurden mit einer Lösung von 7,5 g RhCl₃ x H₂O in 51 ml destilliertem Wasser gleichmäßig getränkt. Der mit Cer, Mangan und Rhodium beaufschlagte Träger wurde im Warmlufttrockner bei 120 °C getrocknet.

4,5 g NaOH und 4,5 g K₂SO₄ wurden in 55 ml destilliertem Wasser gelöst Mit dieser Lösung wurden 170 g des mit Cer, Mangan und Rhodium beaufschlagten Trägers gleichmäßig getränkt und abschließend im Warmlufttrockner bei 120 °C getrocknet bis eine Ablufttemperatur von 110 °C erreicht war.

Der erhaltene Trägerkatalysator enthielt 0,91 Gew.-% Rhodium, 2,16 Gew.-% Mangan und 2,27 Gew.-% Cer, bezogen auf das Gesamtgewicht des Trägerkatalysators.

Der Chrom-Gehalt des Trägerkatalysators betrug weniger als 20 ppm, bezogen auf das Gesamtgewicht des Trägerkatalysators (ermittelt mittels Röntgenfluoreszenzanalyse, Spektrometersoftware SpectraPlus, S8, Bruker AXS, Karlsruhe, Deutschland).

### Beispiel 2b - Umsetzung des Trägerkatalysators mit Wassersoff

Die Durchführung erfolgte analog Beispiel 1b, mit der Änderung, dass 50 ml (42,2 g) des Trägerkatalysators aus Beispiel 2a eingesetzt wurden.

### Beispiel 2c - Verfahren zur Herstellung von OPP unter Verwendung des Trägerkatalysators

Die Durchführung erfolgte analog Beispiel 1c, mit der Änderung, dass der Trägerkatalysator erhalten nach Beispiel 2b eingesetzt wurde.

Nach 267 Stunden wies das Reaktionsprodukt bei 400 °C Katalysatortemperatur folgende Zusammensetzung auf:

| | |
|---|---|
| ortho-Phenylphenol (OPP): | 79 % |
| Diphenyloxid: | 5.9 % |
| Diphenyl: | 0.7 % |
| Phenol: | 0.4 % |

### Beispiel 3

### Beispiel 3a - Herstellung des Trägerkatalysators

185,7 g eines kugelförmigen Magnesium/Aluminium-Mischoxids mit 2,5 mm Durchmesser, mit einem Magnesiumoxid (MgO)-Gehalt von 7 % und mit einer BET-Oberfläche von 160 - 200 m²/g wurden mit 104,7 ml einer wässrigen Lösung enthaltend 11,52 g Ce(NO₃)₃ x 6 H₂O und 16,98 g Mn(NO₃)₂ x 4 H₂O so lange gerührt, bis die Lösung komplett aufgesaugt war, im Anschluss 18 Stunden bei 120 °C im Warmlufttrockner getrocknet und schließlich 3 Stunden bei 400 °C erhitzt.

250 ml (161 g) des nach dem Erhitzen erhaltenen, mit Cer und Mangan beaufschlagten Katalysatorträgers wurden mit einer Lösung enthaltend 4,41 g RhCl₃ x H₂O in 82,6 ml destilliertem Wasser gleichmäßig getränkt. Der mit Cer, Mangan und Rhodium beaufschlagte Träger wurde im Warmlufttrockner bei 120 °C getrocknet.

4,8 g NaOH und 4,8 g K₂SO₄ wurden in 40 ml destilliertem Wasser gelöst und auf 67,3 ml Gesamtvolumen mit destilliertem Wasser aufgefüllt. Mit dieser Lösung wurden 133,6 g des mit Cer, Mangan und Rhodium beaufschlagten Trägers gleichmäßig getränkt und abschließend im Warmlufttrockner bei 120 °C getrocknet bis eine Ablufttemperatur von 110 °C erreicht war.

Der erhaltene Trägerkatalysator enthielt 1,12 Gew.-% Rhodium, 2,0 Gew.-% Mangan und 2,0 Gew.-% Cer, bezogen auf das Gesamtgewicht des Trägerkatalysators.

Der Chrom-Gehalt des Trägerkatalysators betrug weniger als 20 ppm, bezogen auf das Gesamtgewicht des Trägerkatalysators (ermittelt mittels Röntgenfluoreszenzanalyse, Spektrometersoftware SpectraPlus, S8, Bruker AXS, Karlsruhe, Deutschland).

### Beispiel 3b - Umsetzung des Trägerkatalysators mit Wassersoff

Die Durchführung erfolgte analog Beispiel 1b, mit der Änderung, dass 50 ml (34,5 g) des Trägerkatalysators aus Beispiel 3a eingesetzt wurden.

### Beispiel 3c - Verfahren zur Herstellung von OPP unter Verwendung des Trägerkatalysators

Die Durchführung erfolgte analog Beispiel 1c, mit der Änderung, dass der Trägerkatalysator erhalten nach Beispiel 3b eingesetzt wurde.

Nach 267 Stunden wies das Reaktionsprodukt bei 400 °C Katalysatortemperatur folgende Zusammensetzung auf:

| | |
|---|---|
| ortho-Phenylphenol (OPP): | 79 % |
| Diphenyloxid: | 3.3 % |
| Diphenyl: | 0.3 % |
| Phenol: | 0.3 % |

## Patentansprüche

1. Verfahren zur Herstellung von ortho-Phenylphenol (OPP) durch katalytische Dehydrierung von wenigstens einer Verbindung der Komponente A, die aus vollständig oder teilweise hydriertem OPP besteht, das **dadurch gekennzeichnet ist, dass** ein Trägerkatalysator als Katalysator eingesetzt wird, der Rhodium, Mangan, Cer und einen Träger enthält und weniger als 100 ppm Chrom, bezogen auf das Gesamtgewicht des Trägerkatalysators, enthält.

2. Verfahren gemäß Anspruch 1, **dadurch gekennzeichnet, dass** der Trägerkatalysator
- 0,1 - 5,0 Gew.-%, bevorzugt 0,3 - 2,5 Gew.-%, besonders bevorzugt 0,5 - 1,5 Gew.-% Rhodium,
- 0,025 - 5,0 Gew.-%, bevorzugt 0,1 - 3,0 Gew.-%, besonders bevorzugt 1,0 - 2,5 Gew.-% Mangan und
- 0,025 - 5,0 Gew.-%, bevorzugt 0,1 - 3,0 Gew.-%, besonders bevorzugt 1,5 - 2,5 Gew.-% Cer
enthält, bezogen auf das Gesamtgewicht des Trägerkatalysators.

3. Verfahren gemäß wenigstens einem der Ansprüche 1 bis 2, **dadurch gekennzeichnet, dass** der Trägerkatalysator wenigstens einen weiteren Zusatzstoff enthält.

4. Verfahren gemäß wenigstens einem der Ansprüche 1 bis 3, **dadurch gekennzeichnet, dass** der Trägerkatalysator als wenigstens einen weiteren Zusatzstoff Alkalimetall, bevorzugt ausgewählt aus Natrium, Kalium und Gemischen davon, enthält.

5. Verfahren gemäß wenigstens einem der Ansprüche 1 bis 4, **dadurch gekennzeichnet, dass** der Trägerkatalysator 0,05 - 15 Gew.-%, bevorzugt 0,1 - 10 Gew.-%, Alkalimetall enthält, bezogen auf das Gesamtgewicht des Trägerkatalysators.

6. Verfahren gemäß wenigstens einem der Ansprüche 4 bis 5, **dadurch gekennzeichnet, dass** der Trägerkatalysator als wenigstens einen weiteren Zusatzstoff 0,05 - 3,0 Gew.-%, bevorzugt 0,1 - 2,5 Gew.-%, Schwefel enthält, bezogen auf das Gesamtgewicht des Trägerkatalysators.

7. Verfahren gemäß wenigstens einem der Ansprüche 1 bis 6, **dadurch gekennzeichnet, dass** der Träger des Trägerkatalysators ausgewählt ist aus der Gruppe bestehend aus α-, γ-, θ-, η- und δ- Aluminiumoxiden (Al₂O₃), Magnesium/Aluminium-Mischoxiden, Kieselgel, Kieselgur, Montmorilloniten, Bimsstein und Aktivkohle, bevorzugt aus der Gruppe bestehend aus γ- Aluminiumoxid und Magnesium/Aluminium-Mischoxiden.

8. Verfahren gemäß wenigstens einem der Ansprüche 1 bis 7, **dadurch gekennzeichnet, dass** die BET-Oberfläche des Trägers des Trägerkatalysators 100 - 450 m²/g, bevorzugt 160 - 350 m²/g, besonders bevorzugt 230 - 300 m²/g, beträgt.

9. Verfahren gemäß wenigstens einem der Ansprüche 1 bis 8, **dadurch gekennzeichnet, dass** der Träger des Trägerkatalysators kugelförmig ist, bevorzugt mit einem Durchmesser von 1 - 5 mm, besonders bevorzugt von 2-5 mm, ganz besonders bevorzugt von 2 - 4 mm.

10. Verfahren gemäß wenigstens einem der Ansprüche 1 bis 9, **dadurch gekennzeichnet, dass** der Natrium-Gehalt des Trägers des Trägerkatalysators bis zu 3000 ppm, bevorzugt bis zu 2600 ppm, bezogen auf das Gesamtgewicht des Trägers, beträgt.

11. Verfahren gemäß wenigstens einem der Ansprüche 1 bis 10, **dadurch gekennzeichnet, dass** der Trägerkatalysator weniger als 50 ppm, bevorzugt weniger als 20 ppm Chrom, bezogen auf das Gesamtgewicht des Trägerkatalysators, enthält.

12. Verfahren gemäß wenigsten einem der Ansprüche 1 bis 11, **dadurch gekennzeichnet, dass** die wenigstens eine Verbindung der Komponente A, die aus vollständig oder teilweise hydriertem OPP besteht, ausgewählt ist aus der Gruppe bestehend aus 2-Cyclohexylidencyclohexanon, 2-Cyclohexenylcyclohexanon, 2-Cyclohexylcyclohexanon, 2-Cyclohexylcyclohexanol, 2-Cyclohexylphenol, 3-Cyclohexylphenol, 4-Cyclohexylphenol, 2-Phenylcyclohexanon, 2-Phenylcyclohexanol und Gemischen davon.

## Claims

1. Process for preparing orthophenylphenol (OPP) by catalytic dehydrogenation of at least one compound of the component A which consists of fully or partially hydrogenated OPP, which is **characterized in that** a supported catalyst is used as catalyst, which
contains rhodium, manganese, cerium and a support and contains less than 100 ppm of chromium, based on the total weight of the supported catalyst.

2. Process according to Claim 1, **characterized in that** the supported catalyst contains
- 0.1 - 5.0% by weight, preferably 0.3 - 2.5% by weight, particularly preferably 0.5 - 1.5% by weight, of rhodium,
- 0.025 - 5.0% by weight, preferably 0.1 - 3.0% by weight, particularly preferably 1.0 - 2.5% by weight, of manganese and
- 0.025 - 5.0% by weight, preferably 0.1 - 3.0% by weight, particularly preferably 1.5 - 2.5% by weight, of cerium,
based on the total weight of the supported catalyst.

3. Process according to at least one of Claims 1 to 2, **characterized in that** the supported catalyst contains at least one further additive.

4. Process according to at least one of Claims 1 to 3, **characterized in that** the supported catalyst contains alkali metal, preferably selected from among sodium, potassium and mixtures thereof, as at least one further additive

5. Process according to at least one of Claims 1 to 4, **characterized in that** the supported catalyst contains 0.05 - 15% by weight, preferably 0.1 - 10% by weight, of alkali metal, based on the total weight of the supported catalyst.

6. Process according to at least one of Claims 4 to 5, **characterized in that** the supported catalyst contains 0.05 - 3.0% by weight, preferably 0.1 - 2.5% by weight, of sulphur, based on the total weight of the supported catalyst as at least one further additive.

7. Process according to at least one of Claims 1 to 6, **characterized in that** the support of the supported catalyst is selected from the group consisting of α-, γ-, θ-, η- and δ-aluminium oxides (Al₂O₃), magnesium-aluminium mixed oxides, silica gel, kieselguhr, montmorillonites, pumice and activated carbon, preferably from the group consisting of γ-aluminium oxide and magnesium-aluminium mixed oxides.

8. Process according to at least one of Claims 1 to 7, **characterized in that** the BET surface area of the support is 100 - 450 m²/g, preferably 160 - 350 m²/g, particularly preferably 230 - 300 m²/g.

9. Process according to at least one of Claims 1 to 8, **characterized in that** the support of the supported catalyst is spherical , preferably having a diameter of 1 - 5 mm, particularly preferably 2 - 5 mm, very particularly preferably 2 - 4 mm.

10. Process according to at least one of Claims 1 to 9, **characterized in that** the sodium content of the support of the supported catalyst is up to 3000 ppm, preferably up to 2600 ppm, based on the total weight of the support.

11. Process according to at least one of Claims 1 to 10, **characterized in that** the supported catalyst contains less than 50 ppm, preferably less than 20 ppm, of chromium, based on the total weight of the supported catalyst

12. Process according to at least any of Claims 1 to 11, **characterized in that** the at least one compound of the component A, which consists of fully or partially hydrogenated OPP, is selected from the group consisting of 2-cyclohexylidenecyclohexanone, 2-cyclohexenylcyclohexanone, 2-cyclohexylcyclohexanone, 2-cyclohexylcyclohexanol, 2-cyclohexylphenol, 3-cyclohexylphenol, 4-cyclohexylphenol, 2-phenylcyclohexanone, 2-phenylcyclohexanol and mixtures thereof.

## Revendications

1. Procédé pour la préparation d'ortho-phénylphénol (OPP) par déshydrogénation catalytique d'au moins un composé du composant A, qui est constitué d'OPP totalement ou partiellement hydrogéné, **caractérisé en ce qu'**un catalyseur sur support est utilisé en tant que catalyseur, qui contient du rhodium, du manganèse, du cérium et un support et qui contient moins de 100 ppm de chrome, par rapport au poids total du catalyseur sur support.

2. Procédé selon la revendication 1, **caractérisé en ce que** le catalyseur sur support contient
- 0,1 à 5,0 % en poids, préférablement 0,3 à 2,5 % en poids, particulièrement préférablement 0,5 à 1,5 % en poids de rhodium,
- 0,025 à 5,0 % en poids, préférablement 0,1 à 3,0 % en poids, particulièrement préférablement 1,0 à 2,5 % en poids de manganèse et
- 0,025 à 5,0 % en poids, préférablement 0,1 à 3,0 % en poids, particulièrement préférablement 1,5 à 2,5 % en poids de cérium,
par rapport au poids total du catalyseur sur support.

3. Procédé selon au moins l'une des revendications 1 et 2, **caractérisé en ce que** le catalyseur sur support contient au moins un additif supplémentaire.

4. Procédé selon au moins l'une des revendications 1 à 3, **caractérisé en ce que** le catalyseur sur support contient un métal alcalin, préférablement choisi parmi le sodium, le potassium et des mélanges correspondants, en tant qu'au moins un additif supplémentaire.

5. Procédé selon au moins l'une des revendications 1 à 4, **caractérisé en ce que** le catalyseur sur support contient 0,05 à 15 % en poids, préférablement 0,1 à 10 % en poids, de métal alcalin, par rapport au poids total du catalyseur sur support.

6. Procédé selon au moins l'une des revendications 4 et 5, **caractérisé en ce que** le catalyseur sur support contient, en tant qu'au moins un additif supplémentaire, 0,05 à 3,0 % en poids, préférablement 0,1 à 2,5 % en poids de soufre, par rapport au poids total du catalyseur sur support.

7. Procédé selon au moins l'une des revendications 1 à 6, **caractérisé en ce que** le support du catalyseur sur support est choisi dans le groupe constitué par des oxydes d'aluminium (Al₂O₃) de type α, γ, θ, η et δ, des oxydes mixtes de magnésium/aluminium, un gel de silice, un kieselguhr, des montmorillonites, de la pierre ponce et du charbon actif, préférablement dans le groupe constitué par de l'oxyde d'aluminium de type γ et des oxydes mixtes de magnésium/aluminium.

8. Procédé selon au moins l'une des revendications 1 à 7, **caractérisé en ce que** la surface BET du support du catalyseur sur support est de 100 à 450 m²/g, préférablement de 160 à 350 m²/g, particulièrement préférablement de 230 à 300 m²/g.

9. Procédé selon au moins l'une des revendications 1 à 8, **caractérisé en ce que** le support du catalyseur sur support est sphérique, préférablement doté d'un diamètre de 1 à 5 mm, particulièrement préférablement de 2 à 5 mm, tout particulièrement préférablement de 2 à 4 mm.

10. Procédé selon au moins l'une des revendications 1 à 9, **caractérisé en ce que** la teneur en sodium du support du catalyseur sur support est de jusqu'à 3 000 ppm, préférablement jusqu'à 2 600 ppm, par rapport au poids total du support.

11. Procédé selon au moins l'une des revendications 1 à 10, **caractérisé en ce que** le catalyseur sur support contient moins de 50 ppm, préférablement moins de 20 ppm de chrome, par rapport au poids total du catalyseur sur support.

12. Procédé selon au moins l'une des revendications 1 à 11, **caractérisé en ce que** l'au moins un composé du composant A, qui est constitué d'OPP totalement ou partiellement hydrogéné, est choisi dans le groupe constitué par la 2-cyclohexylidènecyclohexanone, la 2-cyclohexénylcyclohexanone, la 2-cyclohexylcyclohexanone, le 2-cyclohexylcyclohexanol, le 2-cyclohexylphénol, le 3-cyclohexylphénol, le 4-cyclohexylphénol, la 2-phénylcyclohexanone, le 2-phénylcyclohexanol et des mélanges correspondants.
